# EUROPEAN PATENT APPLICATION

(11) **EP 4 260 849 A1**
(43) Date of publication of application: **18.10.2023**
(21) Application number: 21854670.3
(22) Date of filing: 10.12.2021
(51) Int. Cl.: A61K 31/045, A61K 31/192, A61P 3/06

(54) **COMPOUND FOR THE REDUCTION OF WHITE ADIPOSE TISSUE AND THE TREATMENT OF OVERWEIGHT AND OBESITY**

(30) Priority: 11.12.2020 ES 202031235
(71) Applicant: Universidad de Granada, E-18071 Granada (ES)
(72) Inventor: LÓPEZ GARCÍA, Luis Carlos, 18071 Granada (ES); HIDALGO GUTIÉRREZ, Agustín, 18071 Granada (ES); BARRIOCANAL CASADO, Eliana, 18071 Granada (ES); GONZÁLEZ GARCÍA, Pilar, 18071 Granada (ES); DÍAZ CASADO, Mª Elena, 18071 Granada (ES)
(74) Representative: ABG Intellectual Property Law, S.L.
(86) International application number: PCT/ES2021/070881
(87) International publication number: WO 2022/123103

(57) **Abstract**

The present invention relates to β-resorcylic acid for use in reducing the amount of white adipose tissue. The inventors have discovered that β-resorcylic acid causes a selective reduction of white adipose tissue without affecting skeletal muscle mass. The present invention thereby allows the therapeutic use of β-resorcylic acid in preventing the accumulation of white adipose tissue or in reducing white adipose tissue. The present invention also relates to a pharmaceutical composition, to the non-therapeutic use of β-resorcylic acid, and to a nutraceutical composition or functional food, dietary product, or nutritional supplement.

## Description

### Field of the Invention

The present invention is comprised in the field of biomedicine and relates to a compound, β-resorcylic acid, for use in reducing the amount of white adipose tissue. Within this field, use of this compound for the treatment of overweight and/or obesity is of particular interest, where said overweight and/or obesity may also be associated with a metabolic syndrome or hepatic steatosis.

### Background of the Invention

The World Health Organization (WHO) defines overweight and obesity as an abnormal or excessive accumulation of white adipose tissue (WAT) that may impair health. Said accumulation of WAT usually occurs as a result of an energy imbalance between calories consumed and calories expended, with worldwide obesity incidence nearly tripling between 1975 and 2016. In fact, in 2016, 39% of adults 18 years or older were overweight and 13% were obese; and over 340 million children and adolescents (aged 5 to 19) were overweight or obese.

Adipose tissue comprises adipocytes (or adipose cells) and is the main energy reserve of the body. Two types of adipose tissue are known, and they are identified based on their morphological and functional characteristics: white adipose tissue and brown adipose tissue. The adipocytes of white adipose tissue are large, round cells (25-200 mm) characterized by a large unilocular lipid droplet made up of triglycerides and other lipids, surrounded by a thin layer of cytoplasm, some mitochondria, and a flat nucleus that is displaced to the periphery. The main functions of white adipose tissue are the accumulation of energy in the form of triacylglycerols, thermal insulation, and the secretion of adipokines (or adipocytokines) which regulate various biological processes in an autocrine, paracrine, and endocrine manner. Adipocytes are also important to maintain glucose and lipid homeostasis in the body.

Overweight and obesity are important risk factors of noncommunicable diseases including, notably, type II diabetes and metabolic syndrome, as well as the consequences thereof which lead to cardiovascular diseases, some types of cancer, and disorders of the locomotor apparatus.

Type II diabetes is an anomaly in blood glucose regulation and nutrient storage related with resistance to the actions of insulin, with the involvement of other hormones and inter- and intracellular mechanisms of action. However, evidence suggests that diabetes characteristic of insulin resistance is but one aspect of a syndrome of metabolic disorders. Hyperglycemia in these patients is often associated with intra-abdominal obesity, elevated plasma concentrations of triglycerides and reduced plasma concentrations of HDL, high blood pressure, systemic inflammation, abnormal fibrinolysis, abnormal vascular endothelial function, and macrovascular disease (coronary, cerebrovascular, and peripheral arterial disease). These anomalies are known as a whole as metabolic syndrome, and in practice patients with this pathology must meet at least three of the following criteria: abdominal obesity (waist circumference exceeding 88 cm for women and 102 cm for men), serum triglycerides above 150 mg/dL, HDL levels below 50 mg/dL for men and 40 mg/dL for women, blood pressure above 130/85 mmHg, and fasting plasma glucose above 100 mg/dL.

Anomalies in the mitochondrial network, structure, and function is one of the pathological mechanisms which contribute to characteristics, consequences, and evolution of insulin resistance, metabolic syndrome, and obesity. A decrease in the number of mitochondria and in the activity of the mitochondrial respiratory chain, as well as a more widespread mitochondrial dysfunction, have been described in the liver and skeletal muscle of patients with type II diabetes. Likewise, a decrease in the levels of Coenzyme Q (CoQ) biosynthesis proteins, as well as in the mitochondrial levels of CoQ, in the muscle and adipose tissue of patients with insulin resistance and of mice in which said insulin resistance is induced through a high fat-high sucrose (HFHS) diet, have been described.

Different pharmacological strategies are being used for the treatment of type II diabetes, metabolic syndrome, or obesity. However, the results are mixed in the affected population due to the effectiveness of treatments and the presence of side effects. These treatments include, among others, metformin, a mitochondrial complex I inhibitor, which can trigger side effects in chronic treatments; sodium-glucose co-transporter inhibitors which stimulate urinary excretion of glucose; GLP-1 receptor agonists and DPP-4 inhibitors which stimulate the incretin system to lower blood glucose; sulfonylureas which stimulate insulin release by the pancreas; and thiazolidinediones which stimulate mitochondrial biogenesis through PPARγ.

The body weight of an individual is roughly represented by 80% lean tissue (or fat-free mass) and 20% adipose tissue (or storage fat). The content of lean tissue is highly heterogeneous and includes bones, muscles, extracellular water, nerve tissue, and cells and tissues other than adipocytes or fat cells. Accordingly, a decrease in body weight may be due to a loss of the content of any of these components, and therefore a decrease in weight cannot be linked to a decrease in fatty tissue.

For example, sarcopenia is a syndrome associated with aging resulting from a gradual decline in skeletal muscle mass. A reduction in body weight occurs when said loss of skeletal muscle mass is triggered (Fried et al., J Gerontol A Biol Sci Med Sci 2001;56: M146-56). A reduction in body weight can also be associated with a reduction in bone density, as occurs in the osteoporosis process (Tirosh et al., J Clin Endocrinol Metab 2015;100(6):2463-7). These examples are clear evidence of weight loss which is not linked to the loss of fatty tissue.

Furthermore, weight loss achieved by both nutritional diets and specific exercises need not be the result of a loss of fatty tissue (Ramirez-Velez et al., Nutrients 2020 Jul 26;12(8):2231). Furthermore, specific diets to lose weight often induce a loss of muscle mass, and to that end intense research activity is being carried out to look for therapeutic options that reduce fatty tissue without losing muscle mass (Verreijen et al., Am J Clin Nutr 2015;101 (2):279-86).

β-resorcylic acid (β-RA) belongs to the hydroxybenzoic acids, a group of natural phenolic compounds found in plants. β-RA is capable of interfering with CoQ metabolism and acting as a therapeutic agent in primary CoQ deficiencies (Hidalgo-Gutiérrez A, Barriocanal-Casado E, Bakkali M, et al. EMBO Mol Med. 2019; 11(1): e9466), a group of rare mitochondrial diseases. Furthermore, it has been described that β-RA reduces the body weight of CAG-*CreER^{T2}*; *Mclk1*^{*Loxp*/}*^{Loxp},* mice which the authors called "control mice". Nevertheless, the reason for said reduction in body weight is not specified (Wang Y, Oxer D, Hekimi S. Nat Commun. 2015; 6:6393).

Document KR 101627546B B1 discloses a pharmaceutical composition for use in food which is useful in the treatment of obesity and comprises a protocatechuic acid (PCA or 3,4-dihydroxybenzoic acid) derivative with lipase inhibiting activity. However, this activity is based only on the experimental data from the inhibition of porcine pancreatic lipase, which has not been completed with studies in animal models.

In another study, research was conducted on a *Schisandra chinensis* pollen extract comprising phenolic compounds including, among others, 2,4-dihydroxybenzoic acid at a low proportion (0.0673 mg/g) with respect to the rest of the analyzed compounds. The authors describe the activity of said extract in preventing non-alcoholic fatty liver disease and in regulating intestinal microbiota in a mouse model with obesity induced by a high lipid diet (Cheng et al., Nutrients 2019; 11:346).

### Brief Description of the Invention

Surprisingly, the inventors have discovered that β-resorcylic acid causes a selective reduction of white adipose tissue without affecting skeletal muscle mass. The present invention therefore allows β-resorcylic acid to be used in the treatment of conditions such as overweight or obesity without side effects on skeletal muscle mass. Furthermore, such effect also extends to the treatment of the preceding conditions associated with metabolic syndrome or hepatic steatosis. Reduction of white adipose tissue entails a reduction in the occurrence of lipid droplets in the liver caused by any of the preceding conditions.

Therefore, in a first aspect, the present invention relates to β-resorcylic acid or pharmaceutically acceptable salts thereof for use in preventing the accumulation of white adipose tissue or in reducing white adipose tissue.

In a second aspect, the present invention relates to a pharmaceutical composition comprising β-resorcylic acid or pharmaceutically acceptable salts thereof, and a pharmaceutically acceptable excipient, for use in preventing the accumulation of white adipose tissue or in reducing white adipose tissue.

The present invention also contemplates preventing the accumulation of white adipose tissue or reducing white adipose tissue in animals, preferably mammals, even more preferably humans, that do not have a disease associated with excess white adipose tissue. Therefore, in a third aspect, the invention relates to the non-therapeutic use of β-resorcylic acid or salts thereof in preventing the accumulation of white adipose tissue or in reducing white adipose tissue.

A fourth aspect of the invention relates to a nutraceutical composition or functional food, dietary product, or nutritional supplement, comprising β-resorcylic acid, characterized in that the β-resorcylic acid is at a concentration greater than 5% w/w.

### Brief Description of the Figures

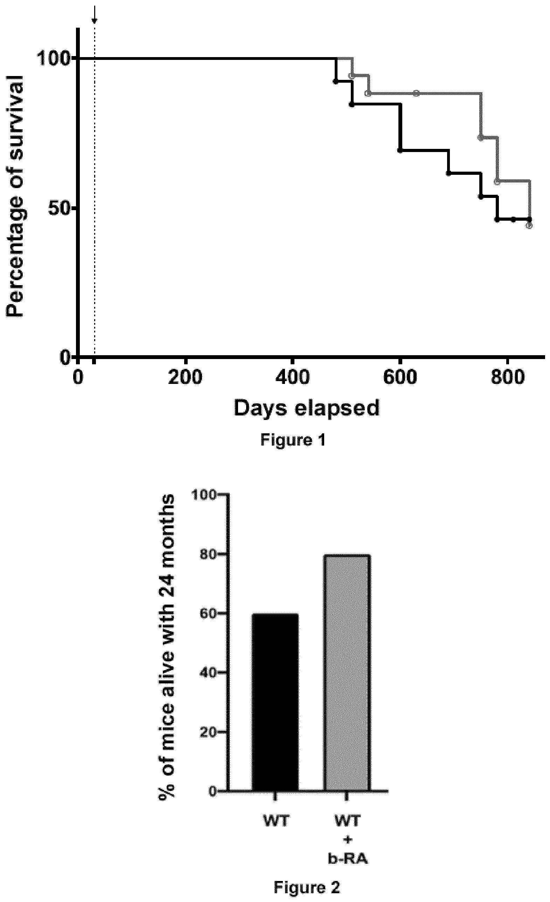

Figure 1. Graph depicting the general survival of wild-type mice during their development. The black line represents wild-type mice with a standard diet, whereas the grey line represents wild-type mice with a standard diet supplemented with β-RA. The arrow pointing to a vertical line means the start of treatment.
Figure 2. Graph depicting survival in the old-age stage (24 months) of wild-type mice during their development. The black bar represents wild-type mice with a standard diet (WT), whereas the grey bar represents wild-type mice with a standard diet supplemented with β-RA (WT+b-RA).

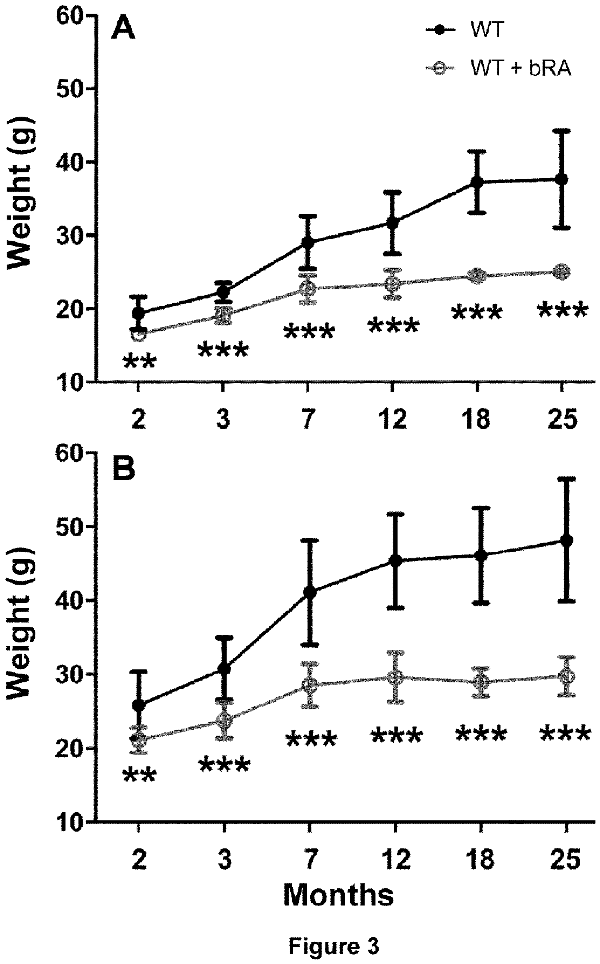

Figure 3. Changes in body weight of wild-type mice during their development. Graph depicting body weight during the development of male wild-type mice (A) and female wild-type mice (B). The black line represents wild-type mice with a standard diet (WT), whereas the grey line represents wild-type mice with a standard diet supplemented with β-RA (WT+b-RA). ** *P*<0.01 vs wild-type with a standard diet; *** *P*<0.001 vs wild-type with a standard diet.

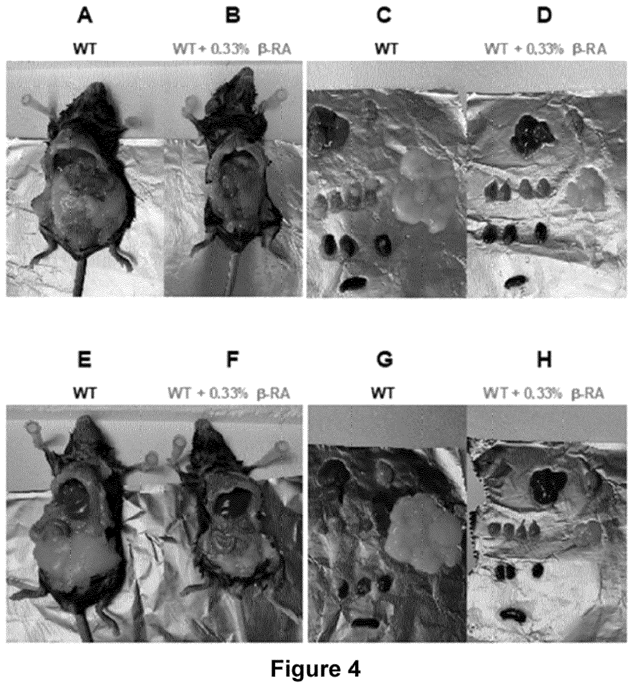

Figure 4. Images representative of the internal aspect and the appearance of the tissues of wild-type mice at the start of old age. (A and C) Male wild-type mouse with a standard diet at 18 months of age; (B and D) Male wild-type mouse with a standard diet supplemented with β-RA at 18 months of age; (E and G) Female wild-type mouse with a standard diet at 18 months of age; (F and H) Female wild-type mouse with a standard diet at 18 months of age.

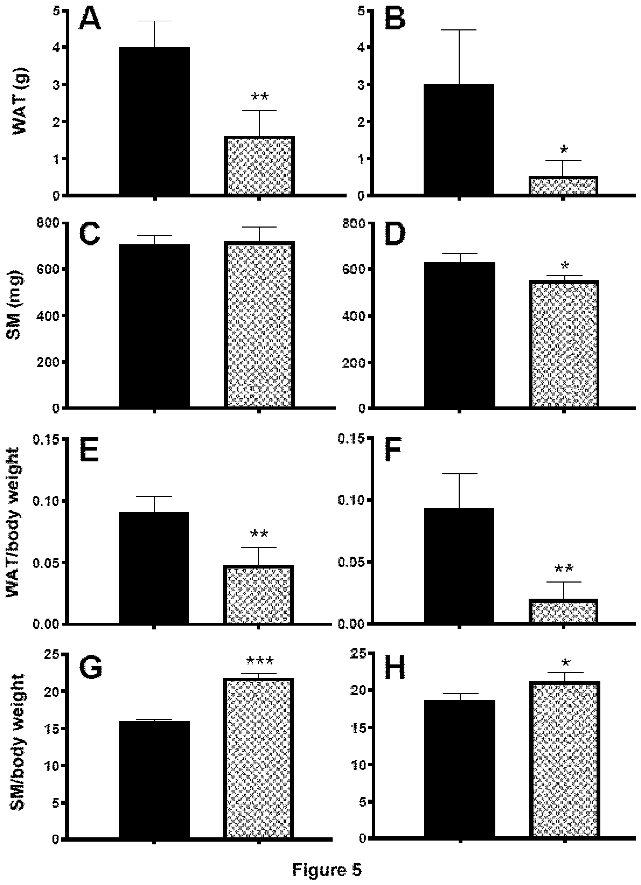

Figure 5. Weight of the abdominal and visceral white adipose tissue and of the skeletal muscles of the hind limbs of wild-type mice at the start of old age (18 months of age). (A-B) Weight of the abdominal and visceral white adipose tissue of male mice (A) and female mice (B); (C-D) weight of the skeletal muscles of the hind limbs of male mice (C) and female mice (D); (E-F) weight of the abdominal and visceral white adipose tissue with respect to the total body weight of male mice (E) and female mice (F); (G-H) weight of the skeletal muscles of the hind limbs with respect to the total body weight of male mice (G) and female mice (H). The black bars represent values in wild-type mice with a standard diet (WT), whereas the grey bars represent values in wild-type mice with a standard diet supplemented with β-RA (WT+b-RA). * *P*<0.05 vs wild-type with a standard diet; ** *P*<0.01 vs wild-type with a standard diet; *** *P*<0.001 vs wild-type with a standard diet.

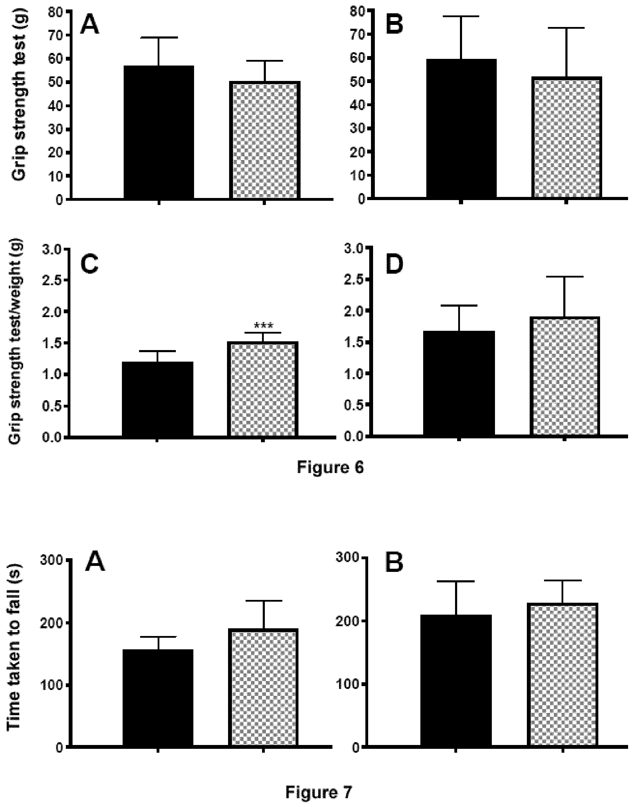

Figure 6. Grip strength test of the hind limbs of wild-type mice at the start of old age (18 months of age). (A and B) Grip strength of male mice (A) and female mice (B); (C and D) Grip strength corrected by the total weight of male mice (C) and female mice (D). The black bars represent values in wild-type mice with a standard diet, whereas the grey bars represent values in wild-type mice with a standard diet supplemented with β-RA. * P<0.05 vs wild-type with a standard diet; ** P<0.01 vs wild-type with a standard diet; *** P<0.001 vs wild-type with a standard diet.
Figure 7. Motor coordination test of wild-type mice at the start of old age (18 months of age). (A and B) Motor coordination test in male mice (A) and female mice (B). The black bars represent values in wild-type mice with a standard diet, whereas the grey bars represent values in wild-type mice with a standard diet supplemented with β-RA. * P<0.05 vs wild-type with a standard diet; ** P<0.01 vs wild-type with a standard diet; *** P<0.001 vs wild-type with a standard diet.

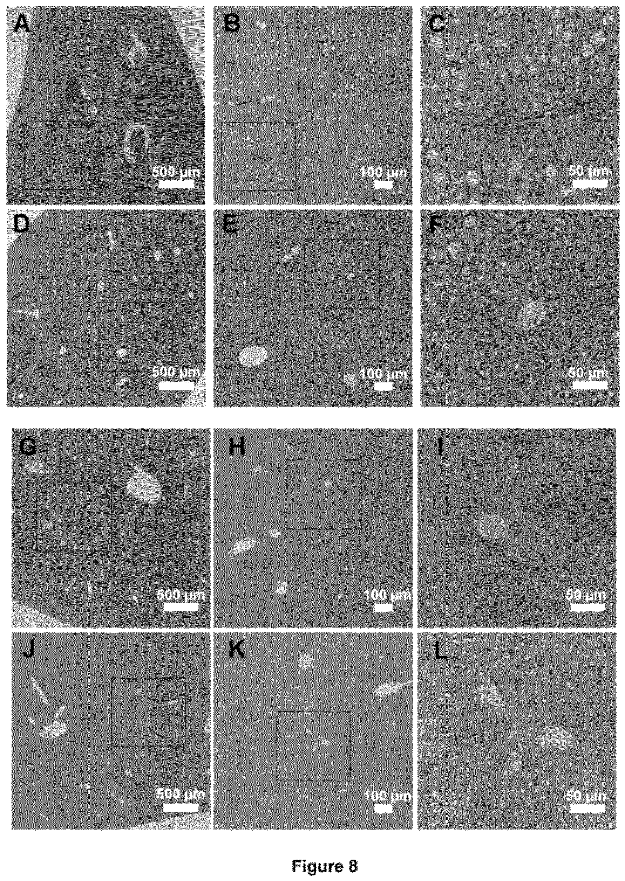

Figure 8. Histological images representative of the liver morphology of wild-type mice at the start of old age (18 months of age). Histological image with H&E staining representative of the liver of a male mouse (A-C) and female mouse (D-F), and of a male mouse (G-I) and female mouse (J-L) supplemented with β-RA.

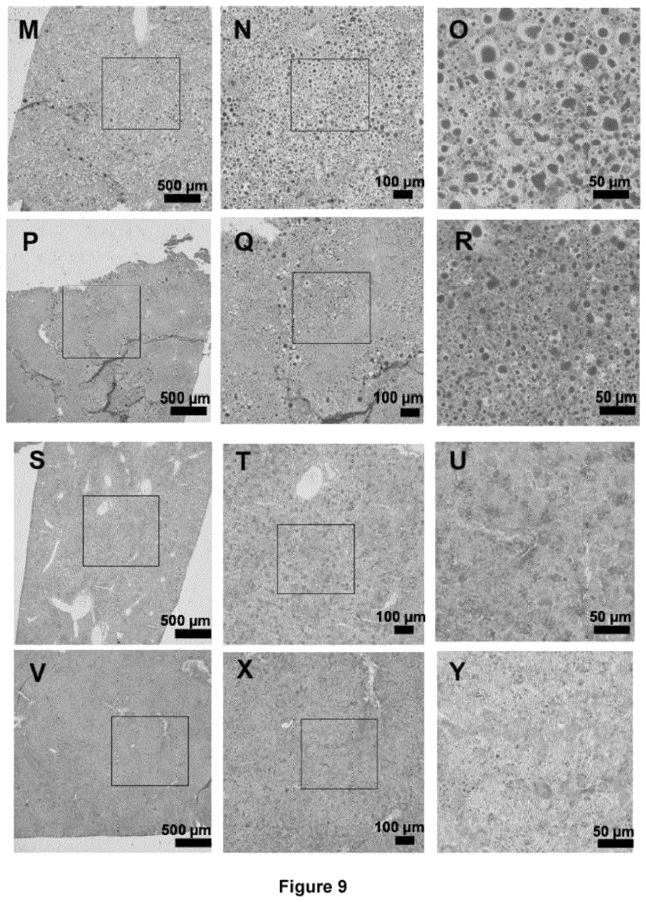

Figure 9. Histological images representative of the liver morphology of wild-type mice at the start of old age (18 months of age). Histological image with "Oil Red O" staining representative of the liver of a male mouse (M-O) and female mouse (P-R), and of a male mouse (S-U) and female mouse (V-Y) supplemented with β-RA.

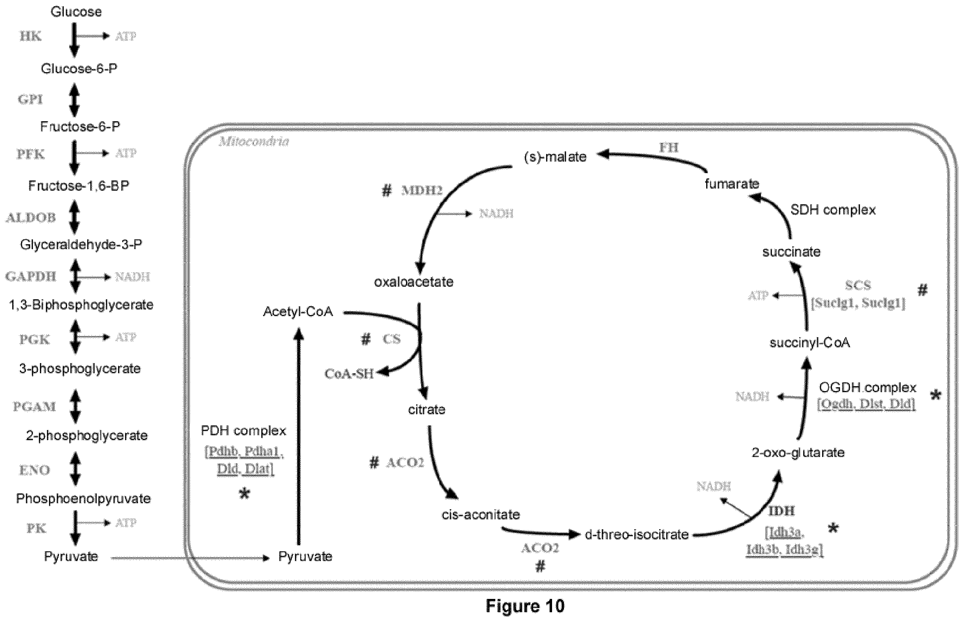

Figure 10. Representation of acetyl-CoA production and the use thereof in the Krebs cycle in a mitochondrion, indicating those proteins that are overexpressed in kidney mitochondria treated with β-RA. The proteins marked with an asterisk (*) indicate that they are overexpressed with the treatment with β-RA, with an increase of more than 1.5 with respect to untreated mice and a value of p<0.01. The proteins marked with a hash (#) indicate that they are overexpressed, without necessarily meeting the two preceding criteria, i.e., an increase of more than 1.5 and a value of p<0.01. The different symbols have the meaning indicated below:

| **Symbol** | **Entrez Gene Name** | **UniProt** | **Expr p-value** | **Expr Fold Change** |
|---|---|---|---|---|
| DLD | dihydrolipoamide dehydrogenase | 008749 | 0.000177 | 1.831 |
| PDHB | pyruvate dehydrogenase E1 subunit beta | Q9D051 | 0.00194 | 1.711 |
| DLAT | dihydrolipoamide S-acetyltransferase | Q8BMF4 | 0.0007 | 1.638 |
| PDHA1 | pyruvate dehydrogenase E1 subunit alpha 1 | P35486 | 0.00207 | 1.61 |
| | | | | |
| DLD | dihydrolipoamide dehydrogenase | 008749 | 0.000177 | 1.831 |
| IDH3A | isocitrate dehydrogenase (NAD(+)) 3 catalytic subunit alpha | Q9D6R2 | 0.00268 | 1.72 |
| IDH3G | isocitrate dehydrogenase (NAD(+)) 3 non-catalytic subunit gamma | P70404 | 0.023 | 1.573 |
| IDH3B | isocitrate dehydrogenase (NAD(+)) 3 non-catalytic subunit beta | Q91VA7 | 0.000942 | 1.727 |
| OGDH | oxoglutarate dehydrogenase | Q60597 | 0.00000664 | 2.05 |
| DLST | dihydrolipoamide S-succinyltransferase | Q9D2G2 | 0.00000204 | 2.316 |
| | | | | |
| CS | citrate syntase | Q9CZU6 | 0.0404 | 1.485 |
| ACO2 | Aconitasa 2 | Q99KI0 | 0.0765 | 1.383 |
| SUCLG1 | Succinate CoA ligase subunit alpha | Q9WUM5 | 0.234 | 1.301 |
| SUCLG2 | Succinate CoA ligase subunit beta | Q9Z2I8 | 0.271 | 1.189 |
| MDH2 | malate dehydrogenase 2 | P08249 | 0.0559 | 1.397 |

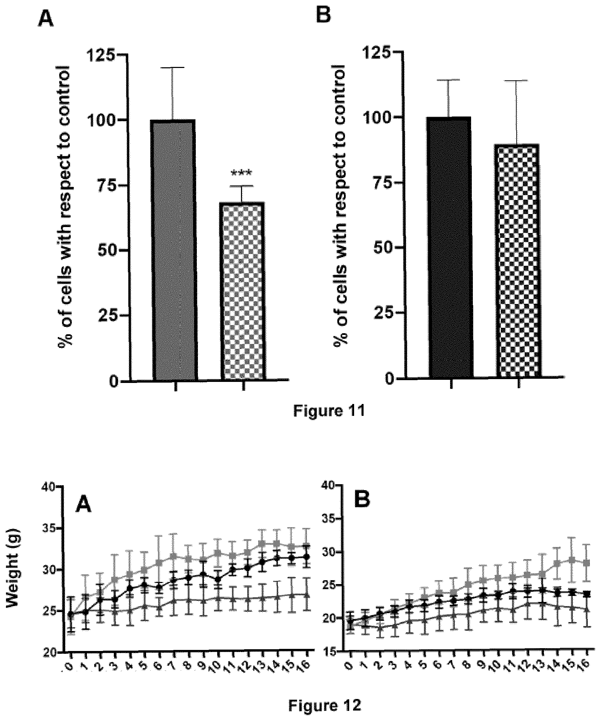

Figure 11. Cell proliferation assay in 3T3L1 preadipocytes and C2C12 myoblasts. (A) Percentage of 3T3L1 cells after 5 days in proliferation conditions; (B) Percentage of C2C12 cells after 5 days in proliferation conditions. *** *P*<0.001 vs untreated cells. The solid bars represent the cells cultured in glucose-rich DMEM-GlutaMAX supplemented with 10% BCS or FBS, 1% non-essential amino acids MEM, and 1% antibiotic/antimycotic, and the checkered bars represent the same cells cultured in the same medium but in the presence of 1 mM of β-RA.
Figure 12. Graph depicting body weight during the development of wild-type mice subjected to a high fat high sucrose (HFHS) diet for 16 weeks for male mice (A) and female mice (B). The line joining circles represents wild-type mice with a standard diet; the line joining squares represents wild-type mice with a HFHS diet; the line joining triangles represents wild-type mice with a HFHS diet treated with β-RA supplement.

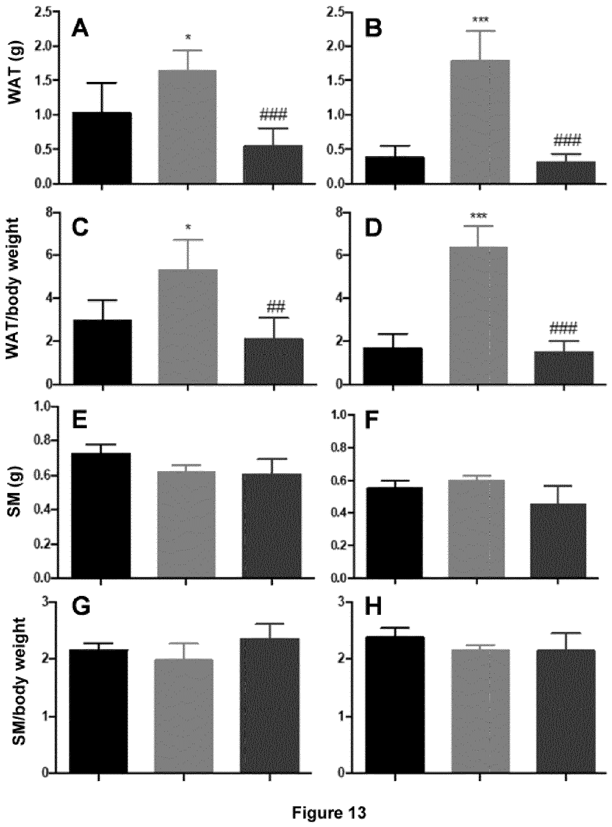

Figure 13. Changes in weight of the abdominal and visceral white adipose tissue of wild-type mice subjected to a high fat high sucrose (HFHS) diet for 16 weeks. (A-B) Weight of the abdominal and visceral white adipose tissue of male mice (A) and female mice (B); (C-D) weight of the abdominal and visceral white adipose tissue with respect to the total body weight of male mice (C) and female mice (D); (E-F) weight of the skeletal muscles of the hind limbs of male mice (E) and female mice (F); (G-H) weight of the skeletal muscles of the hind limbs with respect to the total body weight of male mice (G) and female mice (H). In each graph, the bar on the left represents wild-type mice with a standard diet; the bar at the center represents wild-type mice with a HFHS diet; the bar on the right represents wild-type mice with a HFHS diet treated with β-RA supplement. * *P*<0.05 vs wild-type with a standard diet; ** *P*<0.01 vs wild-type with a standard diet; & *P*<0.05 vs wild-type with a HFHS diet.

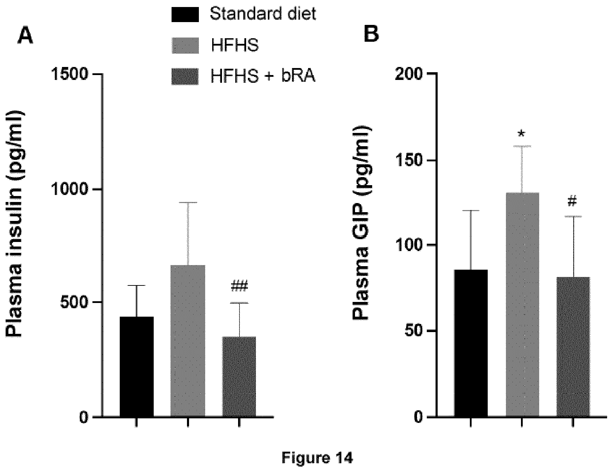

Figure 14. Plasma levels of insulin (A) and gastric inhibitory peptide (GIP) (B) in wild-type mice subjected to a high fat high sucrose (HFHS) diet for 16 weeks. In each graph, the bar on the left represents wild-type mice with a standard diet; the bar at the center represents wild-type mice with a HFHS diet; the bar on the right represents wild-type mice with a HFHS diet treated with β-RA supplement. **P*<0.05 vs wild-type with a standard diet; # *P*<0.05 vs wild-type with a HFHS diet; ## *P*<0.01 vs wild-type with a HFHS diet.

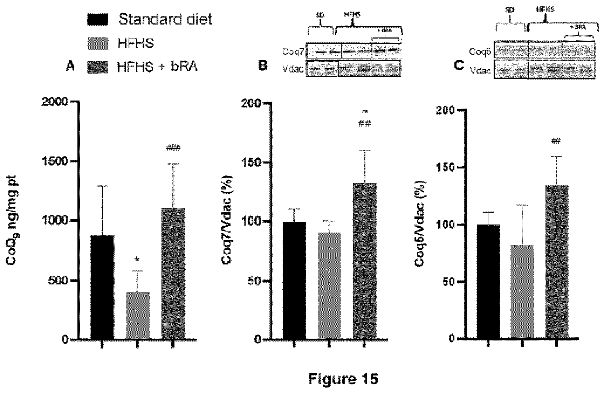

Figure 15. Evaluation of CoQ metabolism in white adipose tissue (WAT) of wild-type mice subjected to a high fat high sucrose (HFHS) diet for 16 weeks. (A) Levels of CoQ9 in WAT; (B) levels of biosynthetic protein Coq7; (C) levels of biosynthetic protein Coq5. In each graph, the bar on the left represents wild-type mice with a standard diet; the bar at the center represents wild-type mice with a HFHS diet; the bar on the right represents wild-type mice with a HFHS diet treated with β-RA supplement. The protein Vdac1 has been used as a mitochondrial fraction load control. * *P*<0.05 vs wild-type with a standard diet; ** *P*<0.01 vs wild-type with a standard diet; ## *P*<0.01 vs wild-type with a HFHS diet; ### *P*<0.001 vs wild-type with a HFHS diet.

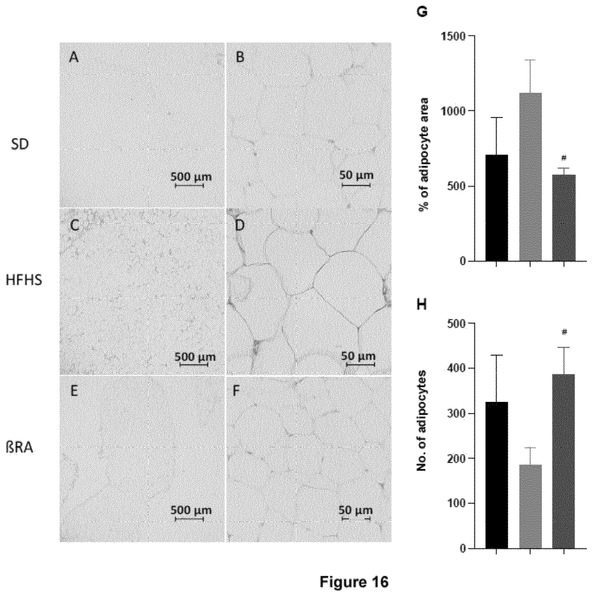

Figure 16. Image of the morphology of the WAT in wild-type mice subjected to a high fat high sucrose (HFHS) diet for 16 weeks. (A-B) Image of WAT in mice with a standard diet; (C-D) image of WAT in mice with a HFHS diet; (E-F) image of WAT in mice with a HFHS diet treated with β-RA; (G) area occupied by each adipocyte; (H) number of adipocytes per area. # *P*<0.05 vs wild-type with a HFHS diet.

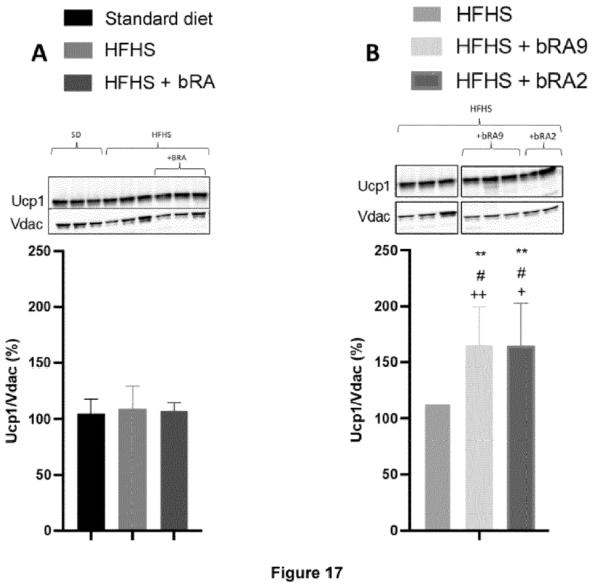

Figure 17. Levels of the thermogenic uncoupling protein Ucp1 in brown adipose tissue (BAT). (A) The graph in which the bar on the left represents wild-type mice with a standard diet; the bar at the center represents wild-type mice with a HFHS diet; the bar on the right represents wild-type mice with a HFHS diet treated with β-RA supplement is shown. (B) The graph in which the bar on the left represents wild-type mice with a HFHS diet; the bar at the center represents wild-type mice with a HFHS diet treated with β-RA supplement after week 9 and sacrificed at week 16; the bar on the right represents wild-type mice with a HFHS diet treated with β-RA supplement after week 9 and sacrificed after two weeks of treatment with β-RA, is shown.

### Detailed Description of the Invention

The present invention focuses on the use of β-resorcylic acid (β-RA) and its salts, including pharmaceutically acceptable salts, for the reduction of white adipose tissue (WAT), and particularly for the treatment of overweight and/or obesity.

One skilled in the art should understand that the different preferred particular embodiments described below for each aspect are not limited to each aspect, but can be applied to different aspects. For example, it should be understood that a particular embodiment of a therapeutic use of the invention is also a particular embodiment of a non-therapeutic use or of a pharmaceutical composition.

The term "β-resorcylic acid" or "β-RA" refers to a compound of formula (I):

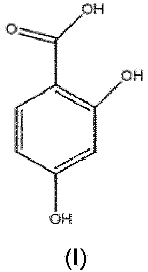

Other equivalent names of this compound are "2,4-dihydroxybenzoic acid", "2,4-DHBA", and "β-resorcinolic acid". In the present application, β-resorcylic acid can also be represented by "b-RA" and one skilled in the art will understand that the text "□-RA" is a typing error that must be interpreted as β-RA.

Unless otherwise indicated, the present invention does not contemplate β-resorcylic acid alone, but also the salts thereof, including pharmaceutically acceptable salts. Non-limiting examples of β-resorcylic acid salts are, for example, lead, monosodium, or disodium salt.

In addition to β-RA, other active compounds are also contemplated in the present invention. In a particular embodiment, unless otherwise indicated, it is considered that "β-RA" may refer to any analog of 2,4-dihydroxybenzoic acid, preferably 4-hydroxybenzoic acid or 3,4-dihydroxybenzoic acid, as well as any of the salts thereof; any functional biological equivalent, and any combination of the foregoing. In a preferred embodiment, β-RA is β-resorcylic acid.

In the present application, unless otherwise indicated, the term "fatty mass" refers to the body weight made up of white adipose tissue.

The term "WT" means "wild-type".

Nutraceutical is defined as any substance which is a food or part of a food and provides medical and/or health benefits, including the prevention and treatment of disease.

### Therapeutic use

According to the World Health Organization (WHO), body mass index (BMI), i.e., weight in kilograms divided by height in meters squared (kg/m²), is an index used for classifying overweight and obesity in adults. The WHO defines overweight as having a BMI equal to or greater than 25, and obesity as having a BMI equal to or greater than 30.

In a first aspect, the present invention relates to β-resorcylic acid or pharmaceutically acceptable salts thereof for use in preventing the accumulation of white adipose tissue or in reducing white adipose tissue.

In a particular embodiment of this aspect, the β-resorcylic acid or pharmaceutically acceptable salts thereof is characterized in that it is at a dose of at least 10, 11, 12, 13, 14, or 15 mg/kg of body weight/day, preferably at least 14 mg/kg of body weight/day, more preferably at least 15 mg/kg of body weight/day, even more preferably at a dose of at least 20 mg/kg of body weight/day.

In another particular embodiment, the β-resorcylic acid or pharmaceutically acceptable salts thereof is characterized in that it is at a dose equal to or less than 60 mg/kg of body weight/day, preferably equal to or less than 55 mg/kg of body weight/day, even more preferably equal to or less than 50 mg/kg of body weight/day.

In another particular embodiment, the β-resorcylic acid or pharmaceutically acceptable salts thereof is characterized in that it is at a dose of between 10 and 60 mg/kg of body weight/day, preferably between 14 and 60 mg/kg of body weight/day, even more preferably between 15 and 60 mg/kg of body weight/day.

The inventors have discovered that β-resorcylic acid causes a selective reduction of white adipose tissue without affecting skeletal muscle mass. Therefore, a preferred embodiment of the present invention contemplates that the use of β-resorcylic acid is characterized in that it maintains the skeletal muscle mass content.

The inventors have also discovered that β-resorcylic acid leads to a reduction in plasma levels of insulin and gastric inhibitory peptide (GIP). Therefore, a particular embodiment of the present invention contemplates that the use of β-resorcylic acid is characterized in that the plasma levels of insulin are reduced. In another particular embodiment, the use of β-resorcylic acid is characterized in that the plasma levels of gastric inhibitory peptide are reduced. In a preferred embodiment, the use of β-resorcylic acid is characterized in that the plasma levels of insulin and gastric inhibitory peptide (GIP) are reduced.

In another particular embodiment of the present invention, the use of β-resorcylic acid is characterized in that the activity of brown adipose tissue (BAT) is stimulated.

The present invention can be used in animals, but it is preferably intended for mammals, preferably humans. Therefore, in a particular embodiment, the use of β-resorcylic acid is characterized in that it is for use in mammals, preferably in humans.

In a particular embodiment of the use of β-resorcylic acid in humans, the use is characterized in that the humans belong to a population aged 30 years or over, 40 years or over, preferably 50 years or over.

In a particular embodiment of the invention, the use of β-resorcylic acid is characterized in that it is for the prevention and/or treatment of overweight, obesity, or conditions associated with metabolic syndrome, insulin resistance, steatosis, preferably hepatic steatosis, or accumulation of lipid droplets in the liver, or for the prevention of heart diseases, prevention of cancer and of strokes.

According to the International Agency for Research on Cancer and based on epidemiological studies, obese and/or overweight people are at a greater risk of developing several types of cancer, such as esophageal adenocarcinoma, colon cancer, breast cancer (in post-menopausal women), endometrial cancer, and kidney cancer.

For this reason, the present invention also contemplates the use of β-resorcylic acid in the prevention of cancer, preferably in the prevention of adenocarcinoma, colon cancer, breast cancer, endometrial cancer, and kidney cancer, preferably in overweight and obese people.

In addition to a greater risk of cancer, obese and/or overweight people are also at a greater risk of other diseases, such as heart diseases, as well as a risk of strokes. Therefore, in a particular embodiment, the present invention also contemplates the use of β-resorcylic acid in the prevention of heart diseases and strokes, preferably in overweight and obese people.

In pathological anatomy, steatosis or fatty change is the name used to refer to the abnormal accumulation of triglycerides in the cytoplasm of parenchymal cells. Steatosis most often occurs in the liver (hepatic steatosis or fatty liver, including non-alcoholic fatty liver), but may also appear in heart, kidney, and other organs.

Metabolic syndrome (also known as syndrome X, plurimetabolic syndrome, insulin resistance syndrome, Reaven's syndrome) is a set of physiological, biochemical, clinical, and metabolic factors which lead to an increased risk of suffering and dying from a cardiovascular disease or from type 2 diabetes mellitus. These factors can be summarized as insulin resistance, excess abdominal fat, atherogenic dyslipidemia, endothelial dysfunction, genetic susceptibility, high blood pressure, hypercoagulable state, and chronic stress. The underlying causes of this syndrome are related to excess calories, sugar, fats, and salt, preferably excess calories, sugar, and fats.

The first aspect of the present invention may also be defined in the following alternative manners:
A method for preventing the accumulation of white adipose tissue or for reducing white adipose tissue which comprises administering an effective amount of β-resorcylic acid or pharmaceutically acceptable salts thereof to an animal in need of same.

Use of β-resorcylic acid or pharmaceutically acceptable salts thereof in the preparation of a medicinal product useful for preventing the accumulation of white adipose tissue or for reducing white adipose tissue.

### Pharmaceutical composition

In a second aspect, the present invention relates to a pharmaceutical composition comprising β-resorcylic acid or pharmaceutically acceptable salts thereof, and a pharmaceutically acceptable excipient, for use in preventing the accumulation of white adipose tissue or in reducing white adipose tissue.

In a particular embodiment, the pharmaceutical composition is characterized in that it maintains the skeletal muscle mass content.

In another particular embodiment, the pharmaceutical composition is characterized in that the use is for the prevention and/or treatment of overweight, obesity, or conditions associated with metabolic syndrome, insulin resistance, steatosis, preferably hepatic steatosis, or accumulation of lipid droplets in the liver, or for the prevention of heart diseases, prevention of cancer and of strokes.

In a particular embodiment of the present invention, the pharmaceutical composition is characterized in that the plasma levels of insulin are reduced. In another particular embodiment of the present invention, the pharmaceutical composition is characterized in that the plasma levels of gastric inhibitory peptide are reduced. In a preferred embodiment, the pharmaceutical composition is characterized in that the plasma levels of insulin and gastric inhibitory peptide (GIP) are reduced.

In another particular embodiment, the pharmaceutical composition is characterized in that it stimulates the activity of brown adipose tissue.

In some embodiments, (β-resorcylic acid can be administered using veterinary or pharmaceutical or nutraceutical compositions including β-resorcylic acid, in the form of a salt, if suitable, or it can be used alone or in the form of a combination with one or more carriers that are compatible and acceptable from veterinary, pharmaceutical, or nutraceutical viewpoint, such as diluents or adjuvants, or with another agent. Remington's The Science and Practice of Pharmacy, 21st edition, A. R. Gennaro, (Lippincott, Williams & Wilkins, Baltimore, MD, 2006) discloses a variety of excipients used in the formulation of pharmaceutical compositions and techniques known for the preparation thereof.

In one embodiment, compositions comprising β-resorcylic acid or a salt thereof, and an acceptable excipient, carrier, or diluent, are provided. The composition can also be in different forms including, but not limited to, oral formulations, injectable formulations, and topical, dermal, or subdermal formulations.

In a preferred embodiment, the composition is an oral composition, i.e., it is formulated in a form suitable for oral use, for example, as dietary supplements, lozenges, chewable products, tablets, hard or soft capsules, emulsions, aqueous or oily suspensions, aqueous or oily solutions, dispersible granules or powders, syrups, or elixirs. The compositions intended for oral use can be prepared according to any method known in the art for manufacturing veterinary, pharmaceutical, or nutraceutical compositions, and such compositions can contain one or more agents selected from the group consisting of sweetening agents, bittering agents, flavoring agents, coloring agents, and preservation agents for the purpose of providing exquisite and acceptable preparations.

In tablet format, the tablets can contain β-resorcylic acid in a mixture with non-toxic pharmaceutically acceptable excipients which are suitable for manufacturing tablets. These excipients can be, for example, inert diluents, such as calcium carbonate, sodium carbonate, lactose, calcium phosphate, or sodium phosphate; granulating and disintegrating agents, for example, cornstarch or alginic acid; binding agents, for example, starch, gelatin, or acacia gum; and lubricating agents, for example, magnesium stearate, stearic acid, or talc. The tablets may not be coated or may be coated by means of known techniques to delay disintegration and absorption in the gastrointestinal tract and thereby provide an action that can be sustained for a longer period.

The formulations for oral use can be a hard gelatin capsule, in which the active ingredient is mixed with an inert solid diluent, for example, calcium carbonate, calcium phosphate, or kaolin. The capsules can also be soft gelatin capsules, in which the active ingredient is mixed with water or miscible solvents such as propylene glycol, PEG, and ethanol, or an oil medium, for example, peanut oil, liquid paraffin, or olive oil.

The compositions can also be in the form of oil-in-water or water-in-oil emulsions. The oily phase can be a vegetable oil, for example, olive oil or peanut oil, or a mineral oil, for example, liquid paraffin or mixtures thereof. Suitable emulsifying agents can be naturally occurring phosphatides, for example, soybean, lecithin, and esters or partial esters derived from fatty acids and hexitol anhydrides, for example, sorbitan monooleate, and condensation products of said partial esters with ethylene oxide, for example, polyoxyethylene-sorbitan monooleate. The emulsions can also contain sweetening agents, bittering agents, flavoring agents, and preservatives.

The aqueous suspensions can contain β-resorcylic acid in a mixture with excipients suitable for manufacturing aqueous suspensions. Such excipients are suspending agents, for example, sodium carboxymethylcellulose, methylcellulose, hydroxypropylmethylcellulose, sodium alginate, polyvinylpyrrolidone, tragacanth gum, and acacia gum; the dispersing or wetting agents can be a naturally occurring phosphatide, for example, lecithin, or condensation products of an alkylene oxide with fatty acids, for example, polyoxyethylene stearate, or condensation products of ethylene oxide with long chain aliphatic alcohols, for example, heptadecaethyleneoxycethanol, or condensation products of ethylene oxide with partial esters derived from fatty acids and a hexitol such as polyoxyethylenesorbitol monooleate, or condensation products of ethylene oxide with partial esters derived from fatty acids and hexitol anhydrides, for example, polyethylene sorbitan monooleate. The aqueous suspensions can also contain one or more preservatives, for example, ethyl or n-propyl p-hydroxybenzoate, one or more coloring agents, one or more flavoring agents, and one or more sweetening agents and/or bittering agents, as mentioned above.

The dispersible granules and powders suitable for the preparation of an aqueous suspension by means of the addition of water provide β-resorcylic acid in a mixture with a dispersing or wetting agent, suspending agent, and one or more preservatives. Suitable dispersing or wetting agents and suspending agents are shown by way of example by means of those already mentioned above. Additional excipients, for example, sweetening agents, bittering agents, flavoring agents, and coloring agents can also be present.

Syrups and elixirs can be formulated with sweetening agents, for example, glycerol, propylene glycol, sorbitol, or sucrose. Such formulations can also contain a demulcent, a preservative, flavoring agent/agents, and coloring agent/agents.

The compositions can be in the form of a sterile injectable aqueous or oleaginous suspension. This suspension can be formulated according to the known technique using the suitable dispersing or wetting agents and suspending agents which have been mentioned above. The sterile injectable preparation can also be a sterile injectable solution or suspension in a non-toxic diluent or solvent acceptable from the parenteral viewpoint, for example, as a solution in 1,3-butanediol. Acceptable vehicles and solvents which can be used include, among others, water, Ringer's solution, and isotonic sodium chloride solution. Co-solvents, such as ethanol, propylene glycol, or polyethylene glycols can also be used. Preservatives, such as phenol or benzyl alcohol can be used.

Furthermore, sterile fixed oils are conventionally used as a solvent or suspending medium. For this purpose, any bland fixed oil including synthetic mono- or diglycerides can be used. Furthermore, fatty acids such as oleic acid are used in the preparation of injectable products.

As a vehicle or diluent, the compositions of the present invention can include vegetable oils such as, but not limited to, soybean oil, peanut oil, castor oil, corn oil, cottonseed oil, olive oil, grapeseed oil, sunflower oil, etc.; mineral oils such as, but not limited to, Vaseline, paraffin, silicone, etc.; aliphatic or cyclic hydrocarbons, or alternatively, for example, medium chain triglycerides (such as C8-C12).

The dosage forms can contain from 0.5 mg to 10 g of β-resorcylic acid. In a particular embodiment, the pharmaceutical composition is administered orally and the β-resorcylic acid is at a dose of between 1 and 60 mg/kg of body weight/day, preferably between 15 and 60 mg/kg of body weight/day.

In a particular embodiment of the invention, the pharmaceutical composition is characterized by an oral administration regimen of between 1 and 3 times a day.

In an embodiment of the invention, β-resorcylic acid is present in a veterinary, pharmaceutical, or nutraceutical composition at a concentration of 0.05 to 100% weight/weight, preferably 0.05 to 95%, 0.05 to 90%, 0.05 to 85%, 0.05 to 80%, 0.05 to 70%, 0.05 to 60%, 0.05 to 50%, 0.05 to 40%, 0.05 to 30%, 0.05 to 20%, or 0.05 to 10% weight/weight.

In a particular embodiment, the pharmaceutical composition is characterized in that it is for use in mammals, preferably humans. Preferably, the humans belong to a population aged 30 years or over, 40 years or over, preferably 50 years or over.

### Non-therapeutic uses

The present invention also contemplates the prevention of the accumulation of white adipose tissue or the reduction of white adipose tissue in animals, preferably mammals, even more preferably humans, in a preferred embodiment, in humans who do not necessarily have a disease associated with excess white adipose tissue such as, for example, people with a body mass index below 25.

Therefore, in a third aspect, the invention relates to the non-therapeutic use of β-resorcylic acid or salts thereof in preventing the accumulation of white adipose tissue or in reducing white adipose tissue.

One skilled in the art will understand that all the preferred particular embodiments described above for therapeutic uses will be applicable for the non-therapeutic uses described in this section, provided that they are not incompatible.

In a particular embodiment of the third aspect, the β-resorcylic acid or salts thereof are provided in a nutraceutical composition comprising a nutritionally acceptable excipient. Preferably, said nutraceutical composition is a functional food, a dietary product, or a nutritional supplement.

### Nutraceutical composition

A fourth aspect of the invention relates to a nutraceutical composition, which can be a functional food, dietary product, or nutritional supplement, comprising β-resorcylic acid, characterized in that the β-resorcylic acid is at a concentration greater than 5% w/w, preferably greater than 10%, greater than 20%, greater than 30%, greater than 40%, greater than 50%, or greater than 60% w/w.

The general expression "nutraceutical composition", which contemplates "functional food", "dietary product", or "nutritional supplement", or simply "food", is used herein in a broad sense and covers foods for human beings, as well as foods for animals (i.e., feed). Preferably, the food is for human consumption. Said food can be in the form of a solution or a solid, depending on the use and/or the mode of application and/or the mode of administration.

A nutraceutical composition can be based on milk or beverages derived from milk such as drinkable yoghurt or regular yoghurt, any other type of regular yoghurt beverage, any other type of beverage, including water, juice made from acidic fruits, or beverages flavored with additional fruits having a pH in the range of 2 to 8, in the form of chocolate, ice-cream, nutrition bars, or any food.

### Particular embodiments

Embodiment 1. A β-resorcylic acid or pharmaceutically acceptable salts thereof for use in preventing the accumulation of white adipose tissue or in reducing white adipose tissue.

Embodiment 2. The β-resorcylic acid for use according to embodiment 1, characterized in that it maintains the skeletal muscle mass content.

Embodiment 3. The β-resorcylic acid for use according to any of embodiments 1 or 2, characterized in that the use is for the prevention and/or treatment of overweight, obesity, or conditions associated with metabolic syndrome, steatosis, preferably hepatic steatosis, or accumulation of lipid droplets in the liver, or for the prevention of heart diseases, prevention of cancer and of strokes.

Embodiment 4. The β-resorcylic acid for use according to any one of embodiments 1 to 3, characterized in that it is for use in mammals.

Embodiment 5. The β-resorcylic acid for use according to any one of embodiments 1 to 4, characterized in that it is for use in humans.

Embodiment 6. The β-resorcylic acid for use according to embodiment 5, characterized in that the humans belong to a population aged 30 years or over, 40 years or over, preferably 50 years or over.

Embodiment 7. A pharmaceutical composition comprising β-resorcylic acid or pharmaceutically acceptable salts thereof, and a pharmaceutically acceptable excipient, for use in preventing the accumulation of white adipose tissue or in reducing white adipose tissue.

Embodiment 8. The pharmaceutical composition for use according to embodiment 7, characterized in that it maintains the skeletal muscle mass content.

Embodiment 9. The pharmaceutical composition for use according to any one of the embodiments 7 or 8, characterized in that the use is for the prevention and/or treatment of overweight, obesity, or conditions associated with metabolic syndrome, steatosis, preferably hepatic steatosis, or accumulation of lipid droplets in the liver, or for the prevention of heart diseases, prevention of cancer and of strokes.

Embodiment 10. The pharmaceutical composition for use according to any one of embodiments 7 to 9, characterized in that it is for use in mammals.

Embodiment 11. The pharmaceutical composition for use according to any one of embodiments 7 to 10, characterized in that it is for use in humans.

Embodiment 12. The pharmaceutical composition for use according to embodiment 11, characterized in that the humans belong to a population aged 30 years or over, 40 years or over, preferably 50 years or over.

Embodiment 13. The pharmaceutical composition for use according to any one of embodiments 7 to 12, characterized in that it is an orally administered pharmaceutical composition.

Embodiment 14. The pharmaceutical composition for use according to any one of embodiments 7 to 13, characterized in that it is administered orally, wherein the β-resorcylic acid is at a dose of between 1 and 60 mg/kg of body weight/day, preferably between 15 and 60 mg/kg of body weight/day.

Embodiment 15. The pharmaceutical composition for use according to any one of embodiments 7 to 14, characterized by an oral administration regimen of between 1 and 3 times a day.

Embodiment 16. Non-therapeutic use of β-resorcylic acid or salts thereof in preventing the accumulation of white adipose tissue or in reducing white adipose tissue.

Embodiment 17. Non-therapeutic use according to embodiment 16, characterized in that it maintains the skeletal muscle mass content.

Embodiment 18. Non-therapeutic use according to any one of embodiments 16 or 17, characterized in that the use is for the prevention and/or reduction of overweight and/or obesity.

Embodiment 19. Non-therapeutic use according to any one of embodiments 16 to 18, characterized in that it is for use in mammals.

Embodiment 20. Non-therapeutic use according to any one of embodiments 16 to 19, characterized in that it is for use in humans.

Embodiment 21. Non-therapeutic use according to embodiment 20, characterized in that the humans belong to a population aged 30 years or over, 40 years or over, preferably 50 years or over.

Embodiment 22. Non-therapeutic use according to any one of embodiments 16 to 21, characterized in that the β-resorcylic acid or salts thereof are provided in a nutraceutical composition comprising a nutritionally acceptable excipient.

Embodiment 23. Non-therapeutic use according to embodiment 22, characterized in that the nutraceutical composition is a functional food, a dietary product, or a nutritional supplement.

Embodiment 24. A functional food, dietary product, or nutritional supplement comprising β-resorcylic acid, characterized in that the β-resorcylic acid is at a concentration greater than 5% w/w.

Embodiment 25. The functional food, dietary product, or nutritional supplement according to embodiment 24, characterized in that the β-resorcylic acid is at a concentration greater than 10% w/w.

Embodiment 26. The functional food, dietary product, or nutritional supplement according to embodiment 24, characterized in that the β-resorcylic acid is at a concentration greater than 20% w/w.

### Examples.

The invention will be illustrated below by means of assays performed by the inventors, clearly demonstrating the usefulness of β-resorcylic acid (indicated in the examples as β-RA) in reducing white adipose tissue (WAT) while maintaining the content and function of skeletal muscles, and therefore in treating overweight and obesity.

For the studies, c57BI6j strain control mice were used under the protocols approved by the Animal Experimentation Ethics Committee of the University of Granada and registered by the competent body, the General Directorate of Agricultural and Livestock Production of the Ministry of Agriculture, Livestock, Fisheries, and Sustainable Development of the Regional Government of Andalusia, under reference numbers 18/02/2019/016 and 16/09/2019/153.

The data was expressed as mean ± standard deviation of 4-10 experiments per group. To compare the differences between the 3 experimental groups, a one-way ANOVA analysis was carried out with a post-hoc Tukey's test. A value of p < 0.05 was considered statistically significant. The survival curve was analyzed through the log-rank (Mantel-Cox) and Gehan-Breslow-Wilcoxon tests.

### Example 1 - Situation of overweight - standard diet

In this example intended for long-term evaluation during the aging process, the animals were kept on a standard rodent diet consisting mainly of carbohydrates (66% carbohydrates, 21% proteins, and 13% fats as energy sources), where these mice accumulate fat as they age, imitating a situation of overweight associated with age.

The experimental treatment was based on the administration of β-RA in the chow feed at a concentration of 0.33% (weight/weight), which represents a dose of 0.25-0.75 g/kg of body weight/day.

The mice were put under treatment after weaning at 21 days of life, with said treatment being maintained for the entire life of the animals. Analyses were performed at 18 months of life, unless another age is indicated, after 17 months of treatment.

### Example 2 - Situation of induced obesity - high fat high sucrose diet

In this example intended for performing a short-term evaluation in an induced metabolic syndrome model, the animals were fed on a high fat high sucrose (HFHS) diet (32% carbohydrates, 14% proteins, and 54% fats as energy sources), where said mice experience a higher fat gain, imitating a state of obesity.

The experimental treatment was based on the administration of β-RA in the chow feed at a concentration of 0.33% (weight/weight), which represents a dose of 0.25-0.75 g/kg of body weight/day.

The mice were put under treatment at two months of life, with said treatment being maintained for 4 months. Analyses were performed at 6 months of life, after 4 months of treatment.

### RESULTS

### Example 3 - Quantification of β-RA levels in mouse tissues

Example 1 describes the administration of β-RA in the chow feed at a concentration of 0.33% (weight/weight), which represents a dose of 0.25-0.75 g/kg of body weight/day. Therefore, β-RA has been chronically administered orally.

The quantification of β-RA was carried out by means of HPLC-UV using an Ultra Aqueous C18 column and a mobile phase consisting of 20 mM potassium phosphate and 15% acetonitrile (pH 2.5). The standard curve consisted of β-RA at a concentration of 5, 25, and 100 µg/mL.

In this example, β-RA levels were determined in order to know the bioavailability of the compound when it is administered orally. It has been observed that β-RA appears in the brain, kidneys, liver, and skeletal muscle, with the kidneys being the tissues having the highest level of the compound (Table 1).

**Table 1. β-RA levels in wild-type mice 3 months of age treated with β-RA incorporated in the standard diet at a concentration of 0.33% (w/w).**

| Brain | Kidney | Liver | Muscle |
|---|---|---|---|
| 1.26 ± 0.58 | 43.02 ± 16.51 | 7.04 ± 2.96 | 20.28 ± 9.39 |

Data expressed as mean ± standard deviation. The results were expressed in ng of β-RA/mg of protein. The untreated mice did not show detectable β-RA levels.

The composition has proven to be effective using a dose of between 0.25 and 0.75 g/kg of weight of the mouse. This may correspond to a dose comprised between 20 and 60 mg/kg of weight in humans according to the formula for dose translation based on body surface area (Reagan-Shaw S, Nihal M, Ahmad N (2008) Dose translation from animal to human studies revisited. FASEB J 22: 659-661). Nevertheless, taking in account the publication by Wang, Smith *et al.* (Wang Y, Smith C, Parboosingh JS, Khan A, Innes M, Hekimi S (2017) Pathogenicity of two COQ7 mutations and responses to 2,4-dihydroxybenzoate bypass treatment. J Cell Mol Med 21: 2329-2343), it could also be predicted that a dose of between 1 mg/kg body weight/day and 16 mg/kg of human body weight /day may also be effective.

### Example 4 - Effect of β-RA on the survival and phenotypic state of mice with a standard diet

Survival and body weight were determined through continuous records throughout the life of the animals. Motor coordination and activity were evaluated through the rotarod test. The muscle strength of the hind limbs was evaluated through the grip strength test. The image and the weight of the tissues were taken immediately after sacrificing and dissecting the animals.

After the chronic administration of β-RA (Example 1), an increase in survival was observed in the old-age stage (18-24 months) (Figures 1 and 2) and a reduction of weight was observed in all the stages of development (Figures 3A and 3B).

The observed body weight reduction was due exclusively to the reduction in the amount of abdominal and visceral fat, with the weight of the skeletal muscles being maintained in the normal range (Figures 4 and 5). In fact, muscle function, determined as muscle strength of the hind limbs, remained unchanged and even slightly increased in reference to the total weight of the animal (Figures 6A-D). Likewise, the motor coordination test has demonstrated a slight upward trend of said capacity in mice treated with β-RA (Figures 7A-B).

This example demonstrates that β-RA allows the state of health to be improved and life prolonged.

### Example 5 - Effect of β-RA on the liver morphology of mice with a standard diet

Overweight or obesity, high levels of fat, insulin resistance, and hyperglycemia are factors frequently associated with aging which favor fat deposition in the liver, known as hepatic steatosis, manifesting clinically as non-alcoholic fatty liver disease (NAFLD).

In this example, photographs have been taken of the animals and tissues after dissection. The tissues were fixed in formalin and embedded in paraffin. Several sections (4 µm thick) were deparaffinized with xylene and stained with hematoxylin and eosin staining (Luna-Sanchez M, Diaz-Casado E, Barca E, Tejada MA, Montilla-Garcia A, Cobos EJ, Escames G, Acuna-Castroviejo D, Quinzii CM, Lopez LC (2015) The clinical heterogeneity of coenzyme Q10 deficiency results from genotypic differences in the Coq9 gene. EMBO Mol Med 7: 670-87). The tissues were also snap-frozen in liquid nitrogen-cooled isopentane (Luna-Sanchez M, Diaz-Casado E, Barca E, Tejada MA, Montilla-Garcia A, Cobos EJ, Escames G, Acuna-Castroviejo D, Quinzii CM, Lopez LC (2015) The clinical heterogeneity of coenzyme Q10 deficiency results from genotypic differences in the Coq9 gene. EMBO Mol Med 7: 670-87). Several sections (8 mm thick) of the frozen tissue were stained with "Oil Red O" stain. The sections were examined at 40-400 x magnifications under a Nikon Eclipse Ni-U microscope, and the images were taken under the same light conditions with NIS-Elements Br software.

Histological staining procedures allow knowing morphological changes and changes in liver fat content, and therefore estimating the presence or absence of lipid droplets (hepatic steatosis) characteristic of non-alcoholic fatty liver.

The inventors have observed that both male and female wild-type mice with a standard diet (Example 1) show clear signs of hepatic steatosis at 18 months of age, represented by the presence of lipid droplets which give an intense signal with "Oil Red O" staining (Figures 8A-F and Figures 9M-R). The onset of said hepatic steatosis is prevented by treatment with β-RA, in both male and female mice (Figures 8G-L and Figures 9S-Y).

β-RA allows modifying the aspect and content of the white adipose tissue (WAT).

### Example 6 - Effect of β-RA on kidney mitochondrial proteome in mice with a standard diet

To check if β-RA has effects on energy metabolism in mitochondria, a study was performed on the mitochondrial proteome of kidneys, because the highest β-RA levels are reached in this tissue (see Table 1).

Each kidney was dissected, and the mitochondrial fraction was then isolated by differential centrifugation. Each pellet was resuspended in 500 mL of HEENK medium with a protease inhibitor. The samples were lysed, digested, and treated prior to injection in UHPLC. After quality control, the samples were injected into a UHPLC 1290 system (Agilent Technologies; Santa Clara, USA) coupled to a Q Exactive HF mass spectrometer (Thermo Scientific; Bremen, Germany) in the previously described conditions (Liu F, Lossl P, Rabbitts BM, Balaban RS, Heck AJR (2018) The interactome of intact mitochondria by cross-linking mass spectrometry provides evidence for coexisting respiratory supercomplexes. Mol Cell Proteomics 17: 216-232). The raw files generated were analyzed using MaxQuant v1.6.10 software (Cox J, Mann M (2008) MaxQuant enables high peptide identification rates, individualized p.p.b.-range mass accuracies and proteome-wide protein quantification. Nat Biotechnol 26: 1367-72). The files generated with MaxQuant were analyzed with Perseus: LFQ data was log2-transformed, and identifications that were present in at least 3 of the 5 replicates were kept for subsequent analyses. For the volcano plot, a P-value threshold of 0.01 and 1 for the LFQ value log2 difference has been established.

The inventors have observed that the changes induced by β-RA in the mitochondrial proteome include, among others, an increase in proteins linked to the metabolic pathways for the production of acetyl-CoA from pyruvate and of the tricarboxylic acid cycle or Krebs cycle (Figure 10), and this may contribute to inducing an increase in the mobilization of substrates as sources for producing energy.

### Example 7 - Effect of β-RA on the proliferative capacity of preadipocytes and myoblasts

It is known that one of the WAT expansion mechanisms is hyperplasia, i.e., the capacity of WAT to recruit new adipocytes proliferated from precursor cells. To check whether the effect of β-RA on WAT may also constitute a direct action on WAT, the proliferative capacity of a preadipocyte cell line (3T3L1 ) subjected to treatment with β-RA was determined in *in vitro* assays, and the results were compared with those obtained in a myoblast cell line (C2C12). This could be used to determine whether the possible effects are specific of a given cell type, excluding those signals and consequences resulting from the physiological context of the *in vivo* studies.

Preadipocyte cell line 3T3L1 and myoblast cell line C2C12 were provided by the Cell Culture Department of the Science Instrumentation Center of the University of Granada. The cells were cultured in glucose-rich DMEM-GlutaMAX supplemented with 10% BCS or FBS, 1% non-essential amino acids MEM, and 1% antibiotic/antimycotic. Treatment with β-RA was administered at a concentration of 1 mM for 5 days. A control group was also treated with vehicle (DMSO) at the same concentration (Luna-Sanchez *et al.,* 2015). The proliferation assay was performed with Vybrant^{®} MTT Cell Proliferation Assay Kit (ThermoFisher Scientific), following the indications of the manufacturing company.

The inventors have observed that, *in vitro,* β-RA induces an inhibition of 3T3L1 preadipocyte proliferation (Figure 11A), whereas the same compound did not have a significant effect on the proliferative capacity of C2C12 myoblasts (Figure 11B).

### Example 8 - Effect of β-RA on the survival and phenotypic state of mice with a high fat high sucrose diet

Survival and body weight were determined through continuous records throughout the life of the animals. Motor coordination and activity were evaluated through the rotarod test. The muscle strength of the hind limbs was evaluated through the grip strength test. The image and the weight of the tissues were taken immediately after sacrificing and dissecting the animals.

After administering β-RA to mice with a HFHS diet (Example 2), an increase in weight was observed in the mice in comparison with those subjected to a standard diet (Example 4) and supplementing with β-RA counteracted said increase in weight (Figures 12A-B), due exclusively to the reduction of abdominal and visceral fat (Figures 12C-F). Likewise, said changes do not cause any changes in the skeletal muscle mass (Figures 12G-J). This shows that β-RA is capable of preventing the accumulation of abdominal and visceral fat regardless of diet, i.e., whether the diet is richer in carbohydrates, as in Example 1 (and Example 4), or richer in fats, as in Example 2 (and the present example).

The diet with β-RA allowed modifying the quantitative content of the tissues, particularly of white adipose tissue (WAT), improving motor and muscle functions.

### Example 9 - Endocrine, molecular, and morphological effects of β-RA in mice with a high fat high sucrose diet

Plasma levels of insulin and gastric inhibitory peptide (GIP), also called glucose-dependent insulinotropic peptide, given that both of them tend to increase in cases of obesity with insulin resistance, were determined. The data showed an increase in insulin and GIP in mice fed with a HFHS diet. The administration of β-RA to mice with a HFHS diet reduced the plasma levels of insulin (Figure 14A) and GIP (Figure 14B), normalizing said values.

At the level of WAT, CoQ metabolism was evaluated by determining the levels of the major form of CoQ in rodents, i.e., CoQ₉, as well as the levels of two limiting enzymes in the CoQ biosynthesis pathway, i.e., the hydroxylase Coq7 and the methyltransferase Coq5. The results show a reduction of the levels of CoQ₉ in WAT of mice fed with a HFHS diet, whereas treatment with β-RA increased said levels, achieving the normalization thereof (Figure 15A). This data is consistent with the increase in the levels of Coq7 (Figure 15B) and Coq5 (Figure 15C) induced by treatment with β-RA. These molecular changes translate into morphological changes observed with hematoxylin and eosin staining. The results show that a HFHS diet induces hypertrophy of white adipocytes (Figures 16C-D), when compared with the adipocytes of WAT of mice fed with a standard diet (Figures 16A-B). Treatment with β-RA prevents hypertrophy of WAT (Figures 16C-D). As a result, the area occupied by adipocytes after treatment with a HFHS diet is larger than after treatment with the standard diet, with the number of adipocytes being smaller. Treatment with β-RA normalizes both the area of the adipocytes (Figure 16G) and the number thereof (Figure 16H).

Given that BAT can contribute to the use of energy resources, and therefore of WAT, to produce heat, the levels of the thermogenic uncoupling protein Ucp1 were quantified. The results did not show any changes in the levels of Ucp1 in mice fed with a HFHS diet or treated with β-RA. Nevertheless, if treatment with β-RA is started in already obese mice after 9 weeks with a HFHS diet, induction of Ucp1 is indeed observed both 7 weeks and 2 weeks after starting treatment.

As a whole, the present invention describes a therapy consisting of the delivery of β-RA for reducing WAT with the content and function of skeletal muscles being maintained, and said therapy is therefore useful for the application thereof in overweight and obesity.

## Claims

1. A β-resorcylic acid or pharmaceutically acceptable salts thereof for use in preventing the accumulation of white adipose tissue or in reducing white adipose tissue.

2. The β-resorcylic acid for use according to claim 1, **characterized in that** it is at a dose of at least 14 mg/kg of body weight/day, preferably at least 15 mg/kg of body weight/day.

3. The β-resorcylic acid for use according to any of claims 1 or 2, **characterized in that** it maintains the skeletal muscle mass content.

4. The β-resorcylic acid for use according to any of claims 1 to 3, **characterized in that** the use is for the prevention and/or treatment of overweight, obesity, or conditions associated with metabolic syndrome, insulin resistance, steatosis, preferably hepatic steatosis, or accumulation of lipid droplets in the liver, or for the prevention of heart diseases, prevention of cancer and of strokes.

5. The β-resorcylic acid for use according to any of claims 1 to 4, **characterized in that** it is for use in mammals.

6. The β-resorcylic acid for use according to any of claims 1 to 5, **characterized in that** it is for use in humans.

7. The β-resorcylic acid for use according to claim 6, **characterized in that** the humans belong to a population aged 30 years or over, 40 years or over, preferably 50 years or over.

8. The β-resorcylic acid for use according to any of claims 1 to 7, **characterized in that** the plasma levels of insulin and/or gastric inhibitory peptide (GIP) are reduced.

9. The β-resorcylic acid for use according to any of claims 1 to 8, **characterized in that** the activity of brown adipose tissue is stimulated.

10. A pharmaceutical composition comprising β-resorcylic acid or pharmaceutically acceptable salts thereof, and a pharmaceutically acceptable excipient, for use in preventing the accumulation of white adipose tissue or in reducing white adipose tissue.

11. The pharmaceutical composition for use according to claim 10, **characterized in that** it maintains the skeletal muscle mass content.

12. The pharmaceutical composition for use according to any one of claims 10 or 11, **characterized in that** the use is for the prevention and/or treatment of overweight, obesity, or conditions associated with metabolic syndrome, insulin resistance, steatosis, preferably hepatic steatosis, or accumulation of lipid droplets in the liver, or for the prevention of heart diseases, prevention of cancer and of strokes.

13. The pharmaceutical composition for use according to any one of claims 10 to 12, **characterized in that** it is for use in mammals.

14. The pharmaceutical composition for use according to any one of claims 10 to 13, **characterized in that** it is for use in humans.

15. The pharmaceutical composition for use according to claim 14, **characterized in that** the humans belong to a population aged 30 years or over, 40 years or over, preferably 50 years or over.

16. The pharmaceutical composition for use according to any one of claims 10 to 15, **characterized in that** the plasma levels of insulin and/or gastric inhibitory peptide (GIP) are reduced.

17. The pharmaceutical composition for use according to any one of claims 10 to 16, **characterized in that** the activity of brown adipose tissue is stimulated.

18. The pharmaceutical composition for use according to any one of claims 10 to 17, **characterized in that** it is an orally administered pharmaceutical composition.

19. The pharmaceutical composition for use according to any one of claims 10 to 18, **characterized in that** it is administered orally, wherein the β-resorcylic acid is at a dose of between 1 and 60 mg/kg of body weight/day, preferably between 15 and 60 mg/kg of body weight/day.

20. The pharmaceutical composition for use according to any one of claims 10 to 19, **characterized by** an oral administration regimen of between 1 and 3 times a day.

21. Non-therapeutic use of β-resorcylic acid or salts thereof in preventing the accumulation of white adipose tissue or in reducing white adipose tissue.

22. Non-therapeutic use according to claim 21, **characterized in that** it maintains the skeletal muscle mass content.

23. Non-therapeutic use according to any one of claims 21 or 22, **characterized in that** the use is for the prevention and/or reduction of overweight and/or obesity.

24. Non-therapeutic use according to any one of claims 21 to 23, **characterized in that** it is for use in mammals.

25. Non-therapeutic use according to any one of claims 21 to 24, **characterized in that** it is for use in humans.

26. Non-therapeutic use according to claim 25, **characterized in that** the humans belong to a population aged 30 years or over, 40 years or over, preferably 50 years or over.

27. Non-therapeutic use according to any one of claims 21 to 26, **characterized in that** the β-resorcylic acid or salts thereof are provided in a nutraceutical composition comprising a nutritionally acceptable excipient.

28. Non-therapeutic use according to claim 27, **characterized in that** the nutraceutical composition is a functional food, a dietary product, or a nutritional supplement.

29. A functional food, dietary product, or nutritional supplement comprising β-resorcylic acid, **characterized in that** the β-resorcylic acid is at a concentration greater than 5% w/w.

30. The functional food, dietary product, or nutritional supplement according to claim 29, **characterized in that** the β-resorcylic acid is at a concentration greater than 10% w/w.

31. The functional food, dietary product, or nutritional supplement according to claim 29, **characterized in that** the β-resorcylic acid is at a concentration greater than 20% w/w.
